(19) Europäisches Patentamt
European Patent Office
Office européen des brevets

(11) **EP 1 846 448 B1**

(12) **EUROPÄISCHE PATENTSCHRIFT**


(45) Veröffentlichungstag und Bekanntmachung des Hinweises auf die Patenterteilung:
**22.07.2009 Patentblatt 2009/30**

(21) Anmeldenummer: **06706615.9**

(22) Anmeldetag: **03.02.2006**

(51) Int Cl.:
***C07K 14/705*** *(2006.01)* ***C07K 16/28*** *(2006.01)*

(86) Internationale Anmeldenummer:
**PCT/EP2006/000947**

(87) Internationale Veröffentlichungsnummer:
**WO 2006/082066 (10.08.2006 Gazette 2006/32)**


(54) **DIAGNOSE VON ALLERGISCHEN ERKRANKUNGEN, ATOPISCHEN ERKRANKUNGEN UND/ ODER AUTOIMMUNERKRANKUNGEN DURCH NACHWEIS VON AUTOANTIKÖRPERN GEGEN CD28 IN HUMANEM SERUM**

DIAGNOSIS OF ALLERGIC COMPLAINTS, ATOPIC DISEASES AND/OR AUTO-IMMUNE DISEASES BY THE IDENTIFICATION OF ANTIBODIES AGAINST CD28 IN HUMAN SERUM

DIAGNOSTIC DE MALADIES ALLERGIQUES, DE MALADIES ATOPIQUES ET/OU DE MALADIES AUTO-IMMUNES PAR MISE EN EVIDENCE D'AUTO-ANTICORPS ANTI-CD28 DANS LE SERUM HUMAIN


(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **07.02.2005 DE 102005006217**

(43) Veröffentlichungstag der Anmeldung:
**24.10.2007 Patentblatt 2007/43**



(73) Patentinhaber: **Universitätsklinikum Hamburg-Eppendorf**
**20246 Hamburg (DE)**

(72) Erfinder:
- **NEUBER, Karsten**
  **22609 Hamburg (DE)**
- **MÄHNSS, Birgit**
  **25335 Elmshorm (DE)**
- **HÜBNER, Christian**
  **07749 Jena (DE)**

(74) Vertreter: **UEXKÜLL & STOLBERG**
**Patentanwälte**
**Beselerstrasse 4**
**22607 Hamburg (DE)**



(56) Entgegenhaltungen:
**WO-A-96/08722** **WO-A-02/066059**

- **LE GROS G ET AL: "Immunoregulatory networks in asthma" CLINICAL AND EXPERIMENTAL ALLERGY, Bd. 28, Nr. SUPPL. 5, November 1998 (1998-11), Seiten 92-96, XP002375857 ISSN: 0954-7894**



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).





Printed by Jouve, 75001 PARIS (FR)

**Beschreibung**

Die vorliegende Erfindung betrifft ein Verfahren zur Diagnose von allergischen Erkrankungen, atopischen Erkrankungen und/oder Autoimmunerkrankungen, bei dem man eine Probe von einem Patienten auf das Vorhandensein von anti-CD28 Autoantikörpern untersucht, indem man die Probe mit CD28 in Kontakt bringt, wobei eine Bindung der Autoantikörper an CD28 auf das Vorliegen einer allergischen Erkrankung, atopischen Erkrankung und/oder Autoimmunerkrankung hinweist. Die Erfindung betrifft weiterhin die Verwendung von CD28 zur Diagnose der genannten Erkrankungen und ein zu diesem Zweck gedachtes Kit, das CD28 und markierte anti-Immunglobulin-Antikörper umfaßt.

Die adaptive Immunantwort ist ein wichtiger Bestandteil des körpereigenen Systems zur Abwehr von Infektionen und damit essentiell für die Bewahrung der Gesundheit.

Adaptive Immunantworten werden manchmal jedoch auch durch Antigene ausgelöst, die nichts mit infektiösen Organismen zu tun haben. Derartige unangemessene Immunantworten können zu schweren Erkrankungen führen, u.a. zu Allergien, atopischen Erkrankungen oder Autoimmunität.

Von einer Autoimmunerkrankung spricht man bei einer spezifischen adaptiven Immunreaktion gegen körpereigene Antigene. Man weiß nicht, wodurch Autoimmunreaktionen ausgelöst werden, es spielen allerdings höchstwahrscheinlich sowohl umweltbedingte als auch erblich bedingte Faktoren eine Rolle. Autoimmunerkrankungen führen gewöhnlich zu langfristigen Gewebeschäden, da, die Zellen, die die vom Immunsystem erkannten Selbstantigene exprimieren, zerstört werden können. Daran sind wahrscheinlich vor allem zytotoxische T-Zellen und eine unangemessene Aktivierung von Makrophagen beteiligt. Auch schädliche Antikörperreaktionen können eine Rolle spielen. WO 02/066059 offenbart die Verwendung von blockierenden anti-CD28 Antikörpern zur Behandlung von Autoimmunerkrankungen. Allergien sind von dem Immunsystem vermittelte Reaktionen auf eine im Allgemeinen harmlose, körperfremde Substanz. Allergische Reaktionen treten bei dem allerersten Kontakt mit dem Allergen noch nicht auf. Die erste adaptive Immunreaktion benötigt Zeit und wird in der Regel nicht wahrgenommen. Sobald jedoch gegen das Antigen gerichtete Antikörper oder T-Zellen induziert wurden, führt jeder erneute Kontakt mit diesem Antigen zu Symptomen.

Es gibt verschiedene Typen von Gewebeschädigungen durch Immunreaktionen. Bei Allergien spielen durch IgE-Antikörper vermittelte schnelle allergische Reaktionen, die sogenannte Überempfindlichkeit vom Soforttyp, atopische Allergie oder Atopie die maßgebliche Rolle. Bei Überempfindlichkeiten vom verzögerten Typ sind T-Zell-Antworten die Ursache, die erst nach einem oder zwei Tagen ihr Maximum erreichen.

Die Prävalenz allergischer und atopischer Krankheiten hat in den letzten Jahrzehnten stark zugenommen. Mehr als 20% der Bevölkerung leiden an Allergien vom Soforttyp.

Eine mögliche Erklärung für die Zunahme z.B. der atopischen Dermatitis ist die sogenannte Hygienehypothese, die davon ausgeht, daß atopischen Erkrankungen durch Infektionen in der Kindheit vorgebeugt werden kann. Diese Theorie wird durch bekannte Risikofaktoren für die Entstehung atopischer Erkrankungen, wie kleine Familien oder Leben in Ballungszentren, gestützt. Auch immunologische Hinweise stützen die Hygienehypothese.

Das pathophysiologische Konzept der Atopie basiert heute auf der Annahme, daß allergenspezifische T-Lymphozyten vom Th2-Typ, die bestimmte Zytokine sezernieren, vor allen Dingen Interleukin (IL)-4, IL-5, IL-10, IL-13 und Granulocyte-Macrophage Colony Stimulating Factor (GM-CSF), die Immunreaktion dominieren, während die z.B. Interferon (IFN)-$\gamma$ produzierende Th1 Lymphozyten weniger aktiv sind (Jujo et al., J Allergy Clin Immunol 1992, 90: 323-331). Zytokine wie IL-4, IL-5 und IL-13 sind im Wesentlichen für die Eosinophilie und die vermehrte Produktion von Antikörpern des IgE-Isotyps bei Atopikern verantwortlich (Punnonen et al., Proc Natl Acad Sci USA 1993, 90: 3730-3734). Bei Atopikern läßt sich also eine generelle Verschiebung des Gleichgewichts des Immunsystems von Th1- zu Th2-Antworten feststellen. Generell werden Th1-Antworten eher von Infektionen, wie z.B. bakteriellen Infektionen induziert, während Th2-Antworten z.B. als Reaktionen auf Befall z.B. mit parasitären Würmern ausgelöst werden.

Bei vielen allergischen Erkrankungen ist das auslösende Allergen bekannt oder kann durch Allergietests ermittelt werden. Hierfür werden, insbesondere bei Allergien vom Soforttyp, vor allem sogenannte Skin prick-Tests eingesetzt (Dreborg, J Am Acad Dermatol. 1989, 21:820-821). Mit diesen kann z.B. das auslösende Agens eines Heuschnupfens innerhalb von kurzer Zeit mit großer Sicherheit festgestellt werden. Die Identifizierung des Allergens ermöglicht in diesem Fall oft ein Vermeiden oder Vermindern der Exposition gegenüber dem Allergen, manchmal ist auch eine sogenannte spezifische Immuntherapie möglich, die zu einer Desensibilisierung des Patienten führen kann.

Diese geht mit einer Verringerung der Konzentration an spezifischen IgE und einer Zunahme der Konzentration an spezifischen IgG4 einher (Reid et al., J. Allergy Clin. Immunol. 78: 590-600, 1986), sowie einer Abnahme der Zahl an Mastzellen und Eosinophilen und einer verringerten Ausschüttung von Mediatoren (Varney et al., J. Clin. Invest. 92: 644-651, 1993). Auch eine Induktion von Anergie bei T-Zellen und eine Verschiebung des Cytokinspektrums zu Produktion von IL-10 und Th1-Cytokinen scheint eine Rolle zu spielen (Akdis and Blaser, Allergy 55: 522-530, 2000, Akdis et al., J. Clin. Invest. 102: 98-106, 1998).

Bei vielen Erkrankungen aus dem allergischen Formenkreis jedoch sind die auslösenden Agenzien nicht leicht festzustellen. Bei atopischer Dermatitis oder Asthma z.B. stehen bei der Diagnose im Allgemeinen die Symptome im Vordergrund, und einzelne auslösende Agenzien lassen sich schwer identifizieren. Auch die Therapie ist am allgemeinen auf eine Linderung der Symptome bezogen.

Eine frühe Therapie kann jedoch entscheidend sein, um einer Verschlechterung des Zustandes auf längere Sicht entgegenzuwirken. Beispielsweise kann bei sensibilisierten Kindern mit atopischer Dermatitis die Gabe eines modernen Antihistaminikums eine Verschlechterung verhindern oder die frühzeitige antientzündliche Therapie mit inhalativen Steroiden die Lebensqualität von Kindern mit Asthma bronchiale stark verbessern.

Als Atopie bezeichnet man auch die vererbte Neigung, an einer oder mehrerer der folgenden atopischen Erkrankungen zu leiden, nämlich atopischem Asthma bronchiale, allergischer Rhinokonjunktivitis (Heuschnupfen) oder atopische Dermatitis (atopisches Ekzem). Für die Diagnose einer Atopie gibt es kein einzelnes klinisches Zeichen oder bestimmten Laborwert, sondern es wird im Allgemeinen eine Kombination an klinischen Merkmalen sowie eine Patienten- und Familienanamnese herangezogen.

Bei der Anamnese wird besonders auf das Vorkommen von Ekzemen, allergischem Asthma und allergische Rhinokonjunktivitis abgestellt bzw. auf das frühere Vorkommen, z. B. von Milchschorf, Juckreiz, der durch Schwitzen verstärkt wird, Metallunverträglichkeit oder Photophobie. Klinische Merkmale wie trockene Haut, Entzündungen z.B. in Knie- oder Ellenbogenbeugen oder an bestimmten Stellen im Gesichtsbereich sind wichtige Hinweise auf eine Atopie.

Eine besondere Herausforderung ist die Schwierigkeit der Diagnose, z.B. beim frühkindlichen Asthma bronchiale. Viel zu lange wird das Krankheitsbild oft als rezidivierende obstruktive Bronchitis eingestuft und die Diagnose Asthma bronchiale zu spät gestellt. Neben der Anamnese und dem Nachweis einer frühen Sensibilisierung und/oder gleichzeitiger atopischer Dermatitis wurde in den letzten Jahren auch das Eosinophile kationische Protein (ECP) herangezogen, um Risikokinder (ECP >16 $\mu$g/l) mit frühkindlichem Asthma bronchiale zu erkennen.

Darüberhinaus ist die Bestimmung des ECP auch hilfreich, um die Effektivität einer antientzündlichen Therapie zu kontrollieren. Außer der Bestimmung des ECP ist die Babylungenfunktionsmessung mit Metacholinprovokation eine gute Möglichkeit, bei unklaren Fällen die Diagnose Asthma bronchiale schon im Kleinkindesalter zu sichern. Dabei findet sich keine Korrelation zwischen ECP und der bronchialen Hyperreagibilität, da diese beiden Methoden unterschiedliche Pathomechanismen des Asthma bronchiale detektieren. Eine klare Diagnose sollte bei allen Kleinkindern mit obstruktiver Symptomatik (wheezing) frühzeitig durchgeführt werden, um die Entwicklung eines chronischen Asthma bronchiale zu vermeiden.

In Bezug auf Laboruntersuchungen wird bei allen Erkrankungen des atopischen Formenkreises vor allem die Konzentration an Gesamt-IgE-Antikörpern im Blut festgestellt. Eine erhöhte Konzentration deutet auf eine Atopie bzw. Allergie hin.

Bei der Bestimmung von Gesamt-IgE werden im Labor unterschiedliche immunologische Methoden verwendet. Die Ergebnisse werden in IU/ml (Internationale Einheiten) oder KU/l angegeben.

Die Konzentration an Gesamt-IgE wird z.B. häufig im ELISA (Enzyme linked immuno-sorbent assay) bestimmt. Dazu wird z.B. in einem sogenannten Sandwich-ELISA ein Träger mit anti-humanen IgE-Antikörpern polyklonalen Ursprungs beschichtet, unspezifische Bindungsstellen werden, z.B. mit BSA (Bovinem Serumalbumin), blockiert. Serum des Patienten wird, z.B. in einer 1:10 Verdünnung, mit dem Träger in Kontakt gebracht, gewaschen und gebundenes IgE mit sekundären Antikörpern, und zwar anti-humanen IgE-Antikörpern (bei einem menschlichen Patienten), detektiert. Diese Antikörper sind im Allgemeinen markiert, z.B. mit einem Enzym wie der Alkalischen Peroxidase oder Horseradish (Meerrettich) Peroxidase, die eine leicht zu detektierende und quantifizierende Farbreaktion katalysiert. Auch eine Bestimmung der Gesamt-IgE-Konzentration im Blot (Western Blot oder Dot Blot), RIA (Radio Immunosorbent Assay) oder mit Hilfe von magnetischen Kügelchen (beads) als Trägern und fluoreszenzmarkierten sekundären Antikörpern ist möglich.

Bei Allergikern ist das Gesamt-IgE im Vergleich zu Nichtallergikern häufig erhöht, es gibt jedoch eine Überschneidung bei der Verteilung der IgE-Werte. Als Anhaltspunkt gilt:

- Bei Werten von weniger als 20 IU/ml (bzw. KU/1) ist eine Allergie eher unwahrscheinlich.
- Bei Werten von mehr als 100 IU/ml ist eine Allergie wahrscheinlich.
- Bei Werten zwischen 20 und 100 IU/ml kann eine klare Entscheidung anhand des Gesamt-IgE-Wertes nicht getroffen werden.

Aufgrund dieser Einschränkungen ergibt sich eine eher orientierende Bedeutung dieses Testes.

Werte von über 100 IU/ml können aber, z.B. bei unklarer Krankengeschichte, einen Hinweis darauf geben, daß die Beschwerden des Patienten möglicherweise auf eine Allergie bzw. Atopie zurückzuführen sind.

WO 96/08722 beschreibt, dass T-Zellen, die kein CD28 besitzen, auf Stimuli wie die Restimulierung mit Antigen nicht mehr mit Proliferation reagieren. Es wird daraus geschlossen, dass diese Zellen immunologisch seneszent sind. In einem diagnostischen Test zum Nachweis seneszenter Zellen wird daher mit Hilfe von Antikörpern gegen CD28 getestet, ob Zellen CD28 exprimieren.

Vor diesem Hintergrund stellt sich dem Fachmann daher die Aufgabe, ergänzende Methoden zur Diagnose atopischer und allergischer Erkrankungen sowie von Autoimmunerkrankungen bereitzustellen, die die Sicherheit einer Diagnose erhöhen oder eine Diagnose erst ermöglichen.

Diese Aufgabe wird durch den Gegenstand der Ansprüche gelöst, insbesondere durch die Verwendung von CD28 zur in vitro Diagnose allergischer Erkrankungen, atopischer Erkrankungen und/oder Autoimmunerkrankungen, wobei CD28 ein CD28-Molekül gunzer Länge oder ein extrazellnläres Fragment davon umfaβt. Man kann CD28 für die Untersuchung einer Probe eines Patienten auf das Vorhandensein von anti-CD28 und Autoantikörpern verwenden, indem man die Probe mit CD28 in Kontakt bringt, wobei eine Bindung der Autoantikörper an CD28 auf das Vorliegen einer allergischen Erkrankung, atopischen Erkrankung und/oder Autoimmunerkrankung hinweist.

Die vorliegende Erfindung stellt in diesem Zusammenhang auch ein Verfahren zur Diagnose allergischer Erkrankungen, atopischer Erkrankungen und/oder Autoimmunerkrankungen zur Verfügung, bei dem man eine Probe eines Patienten auf das Vorhandensein von anti-CD28 Autoantikörpern untersucht, indem man die Proben mit CD28 in Kontakt bringt, wobei eine Bindung der Autoantikörper an CD28 auf das Vorliegen einer allergischen Erkrankung, atopischen Erkrankung und/oder Autoimmunerkrankung hinweist, wobei CD28 ein CD28-Molekül gunzer Länge oder ein extrazellnläres Fragment davon umfaβt.

CD28 wird von ruhenden und aktivierten T-Zellen als 44 kDA großes Membranprotein exprimiert. CD28 spielt eine essentielle Rolle bei der Induktion von T-Zell-vermittelten Immunantworten. Die Aktivierung naiver T-Zellen erfordert mindestens zwei rezeptorvermittelte Signale, die durch antigenpräsentierende Zellen (APZ) vermittelt werden.

Das erste Signal ist antigenspezifisch und wird durch die Interaktion zwischen dem "major histocompatibility complex" (MHC) und dem T-Zellrezeptor (TZR) vermittelt. Dieses Signal reicht für die Aktivierung naiver T-Zellen alleine jedoch nicht aus. Es muß hingegen zusätzlich eine Bindung zwischen CD28 auf den T-Lymphozyten und den dazugehörigen Liganden auf den APZ, nämlich CD80 (B7-1) oder CD86 (B7-2) zustande kommen (Appleman et al., Immunol Rev 2003, 192: 161-180; Sharpe et al, Nature Rev 2002, 2: 116-126). Danach beginnen die Zellen zu proliferieren und sich zu Effektorzellen zu differenzieren. Das Molekül CTLA-4 wird ebenfalls auf T-Lymphozyten exprimiert und kann an CD80 und CD86 binden. Im Gegensatz zu CD28 hemmt CTLA-4 die Effektorantwort aktivierter T-Lymphozyten. Wenn z.B. die Regulation der T-Zellen durch CD28 und CTLA-4 gestört ist, können autoreaktive T-Zellen stimuliert werden, die, u.a. bei Autoimmunerkrankungen, eine zentrale pathophysiologische Rolle spielen.

Autoantikörper gegen Oberflächenmoleküle von T-Lymphozyten konnten bei verschiedenen Autoimmunerkrankungen, aber auch bei Infektionen und auch Bluttransfusionen gefunden werden (Osman et al., Clin Rheumatol 1994, 13: 21-27; Swaak, Lymphocytotoxic antibodies. In: Peter J.B., Shoenfeld Y, editors. Autoantibodies. Amsterdam: Elsevier Science, 1996: 478) Das Auftreten dieser Antikörper korreliert bei einigen Krankheiten mit der Krankheitsaktivität (Winfield et al., Clin Immunol 1992; 63: 13-16) und mit funktionellen Störungen der Leukozyten (Winfield et al., Arthritis Rheumatol 1975, 18: 587-594; Morimoto et al., J Clin Invest 1987, 79: 762-768; Tanaka et al., Arthritis Rheum, 1989, 32: 398-405; Sakane et al., J Clin Invest 1979, 63: 954-965; Wernet et al., J Exp Med 1973, 138: 1021-1026; Takeuchi et al., Scand J Immunol 1982, 16: 369-377).

Bisher wurden bei Menschen Autoantikörper gegen CD45, $\beta_2$-Mikroglobulin und gegen HLA-Klasse I-Moleküle gefunden (Mimura et al., J Exp Med 1990, 172: 653-656; Czyzyk et al. Arthritis Rheum 1996, 39: 592-599; Revillard et al., J Immunol 1979, 122: 614-618; Proper et al., Clin Sci (Lond) 1991, 80: 87-93), und bei Tieren Autoantikörper gegen CTLA-4 (Khatlani et al., J Immunother 2003, 26: 12-20). Autoantikörper gegen CD28 sind bisher nicht beschrieben worden (Khatlani et al.; Matsui et al., J Immunol 1999, 162: 4328-4335).

Im Rahmen dieser Erfindung wurde überraschend gefunden, daß das Auftreten von CD28 Autoantikörpern signifikant mit atopischen Erkrankungen, allergischen Erkrankungen sowie Autoimmunerkrankungen assoziiert ist, z.B. mit dem atopischer Dermatitis (Odds-Ratio 25.31 [95% CI (confidence intervall), 5.52-116.11]; ($p<0.0001$), allergischem Asthma und Rhinokonjunktivitis allergica (Odds-Ratio 10.78 [95% CI, 5.39-21.55]; $p<0.0001$) sowie Autoimmunerkrankungen wie z.B. Sklerodermie. Alle anderen Krankheiten, die bei Patienten, deren Seren untersucht wurden, diagnostiziert wurden, waren nicht mit dem Auftreten von CD28 Autoantikörpern korreliert (Abbildung 4, Tabelle 2).

Grundsätzlich zeigt es sich, daß das Vorhandensein von CD28 Autoantikörpern tendenziell mit jüngerem Alter und weiblichem Geschlecht korreliert ist. Um andere Einflussgrößen, wie z.B. das Serum IgE auszuschließen, wurde zusätzlich eine multivariante logistische Regressionsanalyse durchgeführt. Dadurch konnte ein möglicher Einfluß des Alters, des Geschlechts oder des Serum IgE im Serum als Kofaktor statistisch ausgeschlossen werden (Abb. 5, Tabelle 3).

Im Rahmen dieser Erfindung wird als CD28 ein CD28-Molekül ganzer Länge oder ein Fragment davon bezeichnet, das von anti-CD28 Autoantikörpern erkannt werden kann. Bevorzugt handelt es sich dabei um ein extrazelluläres Fragment. Insbesondere umfaßt das extrazelluläre Fragment von CD28 die in CD28 ganzer Länge vorkommenden Aminosäuren mit Ausnahme des intrazellulären Teils und der transmembranen Region. Bevorzugt weist das extrazelluläre Fragment die Sequenz gemäß SEQ ID NO:2 auf. Diese beschreibt die Sequenz eines humanen exztrazellulären Fragmentes.

Die Sequenz von humanem CD28 ganzer Länger ist in SEQ ID NO:1 angegeben. Neben humanen CD28-Molekülen oder Fragmenten davon sind jedoch auch CD28-Proteine oder Fragmente davon aus anderen Spezies im Rahmen dieser Erfindung umfaßt, wie z.B. aus Maus, Ratte, Kaninchen, Meerschweinchen, Hund, Katze, Pferd oder Rind.

Das CD28-Molekül ganzer Länge oder das extrazelluläre Fragment davon können Teil eines Fusionsproteins sein. Bevorzugt umfaßt das Fusionsprotein ferner Glutathion-S-Transferase oder ein Histidin-Tag, wobei dies besonders zur Aufreinigung des rekombinanten Proteins hilfreich ist. Grundsätzlich kann CD28 aus Zellen aufgereinigt oder rekombinant hergestellt sein. Das Fusionsprotein kann einen Ig (Immunglobulin)-Teil umfassen, es ist jedoch bevorzugt, daß dieser nicht in dem Fusionsprotein enthalten ist, so daß Schwierigkeiten mit möglicher Kreuzreaktivität von im Patientenserum vorkommenden Autoantikörpern gegen den Ig-Teil vermieden werden. CD28 kann jedoch aus CD28-Ig-Fusionsmolekülen abgespalten werden, z.B. mit Trypsin.

Die Probe eines Patienten ist bevorzugt eine Blutprobe oder eine Serumprobe. Das erfindungsgemäße Verfahren wird im Allgemeinen *in vitro* durchgeführt.

Bevorzugt ist CD28 an einen Träger gebunden. Ein solcher fester Träger kann z.B. eine ELISA-Platte, ein magnetisches Kügelchen oder eine Blotfolie, z.B. eine Nitrozellulosefolie, sein. Als Träger werden im Rahmen der Erfindung auch Zellen bezeichnet, die CD28 natürlicherweise oder rekombinant an ihrer Oberfläche exprimieren. CD28 kann direkt an den Träger gebunden, oder, z.B. über Antikörper, insbesondere Antikörper gegen einen mit CD28 verbundenen Tag, wie Glutathion-S-Transferase, an einen Träger gekoppelt werden. Diese Antikörper sind keine humanen Antikörper, um Kreuzreaktionen mit sekundären Antikörpern zu verhindern.

In einer bevorzugten Ausführungsform der Erfindung wird die Bindung von anti-CD28 Autoantikörpern an CD28 untersucht, indem man den Träger mit markiertem anti-Immunglobulin-Antikörpern gegen Antikörper der Spezies, der der Patient angehört, kontaktiert, und man die markierten Antikörper nachweist. Beispielsweise kann der Patient ein Mensch sein. In diesem Fall wird bevorzugt humanes CD28 verwendet, und die anti-Immunglobulin-Antikörper sind anti-humane Immunglobulin-Antikörper.

Bevorzugt sind die anti-Immunglobulin-Antikörper spezifisch für Antikörper des IgG-Isotyps, sie können jedoch auch gegen IgG und IGM und/oder IgE reaktiv sein.

Bevorzugt sind die anti-Immunglobulin-Antikörper mit einem Enzym, beispielsweise Alkalischer Phosphatase oder MeerrettichPeroxidase, Biotin, einem radioaktiven Isotop oder einem Fluoreszenzfarbstoff, z.B. Fluoresceinisothiozyanat (FITC) oder Phycoerythrin (PE) markiert.

Die Untersuchung kann in einem Blot, z.B. einem Dotblot oder einem Westernblot, einem ELISA (Enzyme linked immunosorbent assay), einem RIA (Radioimmunoassay), einer FACS (Fluorescence activated cell sorting)-Untersuchung oder auch in einem Flüssigphasendetektionssystem durchgeführt werden. Sind die Träger Zellen, so findet die nachfolgende Untersuchung der Bindung bevorzugt im FACS oder fluoreszenzmikroskopisch statt.

Insbesondere kann das erfindungsgemäße Verfahren oder die erfindungsgemäße Verwendung von CD28 zur Diagnose von Rhinoconjunktivitis allergica (Heuschnupfen) oder allergischem Asthma bronchiale verwendet werden. Eine besonders hohe Korrelation zwischen CD28 Autoantikörpern und Erkrankung findet sich auch bei der atopischen Dermatitis. Aus dem Formenkreis der Autoimmunerkrankungen kann mit Hilfe von anti-CD28-Autoantikörpern insbesondere die Sklerodermie oder Lupus erythematodes, aber auch Rheumatoide Arthritis und Dermatomyositis nachgewiesen werden.

Anti-CD28-Autoantikörper kommen auch bei bullösen Autoimmunerkrankungen der Haut (Pemphigus vulgaris, bullöses Pemphigoid) vor.

Der Nachweis von anti-CD28-Antikörpern kann ohne ergänzende Diagnoseverfahren, insbesondere Laboruntersuchungen, zur Diagnose einer der genannten Erkrankungen verwendet werden. Bevorzugt ist jedoch die Kombination mit anderen Kriterien. Insbesondere spielen selbstverständlich weiterhin die Patienten-und Familienanamnese sowie die klinischen Symptome eine dominante Rolle bei der Diagnose.

Bei weiteren Laboruntersuchungen ist im atopischen und allergischen Formenkreis die Bestimmung der Konzentration an Gesamt-IgE in einer Probe eines Patienten, beispielsweise einer Blutprobe oder Serumprobe, von besonderer Wichtigkeit. Dabei weist eine Konzentration von 100 IU/ml Gesamt-IgE oder mehr auf das Vorliegen einer allergischen Erkrankung und/oder atopischen Erkrankung hin. Eine Konzentration von 20-100 IU/ml Gesamt-IgE läßt keine genaue Aussage in Bezug auf das Vorliegen einer derartigen Erkrankung zu (Sanz ML, Prieto I, Garcia BE, Oehling A. Diagnostic reliability considerations of specific IgE determination. J Invest Allergol Clin Immunol. 1996 May-Jun;6(3): 152-61.). Besonders in diesem Fall ist eine ergänzende Diagnose mit Hilfe von CD28 sinnvoll.

Bei Verdacht auf Asthma ist weiterhin die Kombination des erfindungsgemäßen Diagnoseverfahrens mit Provokationstests, z.B. mit Metacholin, sinnvoll. Auch andere Untersuchungen, wie z.B. auf das Eosinophile kationische Protein (Wolthers OD. Eosinophil granule proteins in the assessment of airway inflammation in pediatric bronchial asthma. Pediatr Allergy Immunol. 2003 Aug;14 (4) : 248-54.), können mit dem erfindungsgemäßen Verfahren kombiniert werden.

Im Rahmen der vorliegenden Erfindung wird weiterhin ein Verfahren zur Diagnose eines besonderen Risikos, einer besonders schweren Erkrankung oder einer besonderen Schwere der Erkrankung zur Verfügung gestellt, da festgestellt wurde, daß die

Konzentration an Autoantikörpern gegen CD28 damit positiv korreliert ist. Ein wichtiger Parameter für die Schwere einer atopischen Erkrankung ist das Serum IgE. An dem untersuchten Patientengut zeigte sich eine Korrelation zwischen Höhe des Serum IgE und der Höhe des Titers der anti-CD28 Autoantikörper.

Ebenfalls wird ein Kit zur Durchführung eines erfindungsgemäßen Verfahrens bereitgestellt. Bevorzugt ist dieses Kit zur Diagnose von allergischen Erkrankungen und/oder atopischen Erkrankungen geeignet und umfaßt CD28 und markierte anti-Immunglobulin-Antikörper.

Bevorzugt handelt es sich um humanes CD28 und markierte anti-humane Immunglobulin-Antikörper, insbesondere anti-humane IgG-Antikörper.

In einer bevorzugten Ausführungsform umfaßt das erfindungsgemäße Kit ferner markierte anti-IgE-Antikörper und ist damit dazu geeignet, diagnostische Tests zur Feststellung von allergischen oder atopischen Erkrankungen sowohl auf Basis der Feststellung der Konzentration an Gesamt-IgE als auch auf Basis des Nachweises von CD28 Autoantikörpern durchzuführen. Das Kit kann weiterhin unmarkierte anti-IgE-Antikörper umfassen, so daß der Test auf Gesamt-IgE als Sandwich-ELISA durchgeführt werden kann. Die unmarkierten Antikörper können polyklonale anti-IgE-Antikörper sein, die markierten anti-IgE-Antikörper können polyklonal oder monoklonal sein. Alternativ können die markierten und die unmarkierten anti-IgE Antikörper beide jeweils monoklonale Antikörper sein, die jedoch gegen ein unterschiedliches Epitop gerichtet sein müssen.

Die in dem Kit enthaltenen markierten Antikörper können mit einem Enzym, z.B. alkalischer Phosphatase oder Meerrettich-Peroxidase, Biotin, einem radioaktiven Isotop oder einem Fluoreszenzfarbstoff, z.B. FITC oder PE, markiert sein.

## BEISPIELE

### <u>Beispiel 1:</u>

### Enzymatische Spaltung des rekombinaten CD28-Ig Fusionsprotein mit Trypsin und Nachweis von Autoantikörpern gegen CD28 im Immunoblot

Das rekombinante CD28-Ig Fusionsprotein (R & D Systems Inc. Minneapolis, USA) wurde in PBS-Puffer (2,7 M NaCl, 54 mM KCl, 87 mM $Na_2HPO_4$, 30 mM $KHPO_4$, pH7, 4) in einer Konzentration von 1 mg/ml gelöst und 100 μl dieser Lösung wurden mit 50 μl Trypsin (10 mg/ml) bei 37°C für 15 Minuten verdaut. Am Ende der Inkubationszeit wurden 1,5 μl Aprotinin (10 mg/ml) und 2,5 μl TLCK (Nα-p-Tosyl-L-lysin-chlormethylketon, 20 mg/ml) hinzugefügt, um die enzymatische Aktivität von Trypsin zu hemmen. Die Lösung kann bis zum weiteren Gebrauch bei -20°C gelagert werden.

Die gelelektrophoretische Auftrennung von Proteinen (SDS-PAGE) wird nach der Methode von Lughtenberg et al. (Lughtenberg, B. (1975), FEBS Lett. 58, 254) durchgeführt. Die Spaltprodukte wurden durch SDS-Gel Elektrophorese (10%iges Gel) mit einem 4% Sammelgel (110 V, 150 Minuten) unter nicht-reduzierenden Bedingungen aufgetrennt. Anschließend wurden die Spaltprodukte bei einer Stromstärke von 50 mA 3 Stunden lang auf PVDF (Polyvinylidenfluorid)-Membranen (Segin-Blot, Biorad, Germany) transferriert. Anschließend wurden die Membranen mit 5% Magermilchpulver für 60 Minuten bei Raumtemperatur blockiert und mit PBS dreimal gewaschen.

Die Sensitivität und Spezifität des Immunblots wurde mit den folgenden Antikörpern kontrolliert: monoklonaler Maus anti-human CD28 Antikörper (R&D Systems, Minneapolis, USA), 1:5.000 in PBS verdünnt, biotinylierter polyklonaler Maus anti-human CD28 Antikörper (R&D Systems, Minneapolis, USA), 1:5.000 in PBS verdünnt, monoklonaler Maus anti-human Fc Antikörper (Dianova, Hamburg, Germany), 1:10.000 in PBS verdünnt, und polyklonaler Kaninchen anti-human IgG Antikörper (Sigma-Aldrich, Steinheim, Germany), 1: 3.500 in PBS verdünnt.

**Fig. 1** zeigt, daß ein Spaltprodukt des CD28-Ig Fusionsprotein eindeutig nur von den Anti-CD28 Antikörper erkannt wird, nicht aber von Antikörpern gegen IgG oder Fc.

Zum Nachweis der Autoantikörper gegen CD28 im Serum wurden die in Streifen geschnittenen PVDF-Membranen in 1:10 verdünnten humanem Serum für 1 Stunde auf einem Schwenktisch bei Raumtemperatur inkubiert. Als Kontrollen dienten spezifische Antiseren gegen humanes Fc (Dianova, Hamburg) und humanes CD28 (R&D, Wiesbaden). Anschließend wurden die Blots wieder dreimal gewaschen und dann für 1 Stunde bei Raumtemperatur mit einem sekundären, AP-konjugierten Antikörper gegen humanes IgG (Fa. Serva, Heidelberg) inkubiert. Die Bindung wurd durch eine enzymatische Farbreaktion (BCIP/NBT, 5-bromo-4-chloro-3-indolyl phosphate/ p-nitroblue tetrazolium chloride) sichtbar gemacht.

Exemplarisch sind die Ergebnisse von Seren, die Autoantikörper gegen CD28 enthalten oder negativ sind, in **Fig. 2** dargestellt. Mit Hilfe des Immunoblots können im Seren spezifische IgG-Antikörper gegen CD28 nachgewiesen werden.

**Beispiel 2:**

**Assoziation von Autoantikörpern gegen CD28 mit verschiedenen Krankheiten**

Insgesamt wurden 268 Seren auf das Vorhandensein von Autoantikörpern gegen CD28 mit dem Verfahren getestet und ausgewertet. Aus dieser Gruppe stammten 72 Seren von gesunden Probanden, 196 Seren stammten von Patienten mit unterschiedlichen Krankheiten. Von jedem Patienten lag das schriftliche Einverständnis zur Blutentnahme aus wissenschaftlichen Gründen vor.

In der Gruppe der gesunden Probanden wurden CD28 Autoantikörper in 8/72 Seren nachgewiesen (11.1%). In der Patientengruppe waren 53/196 Seren (27.04%) positiv. **Tabelle 1** zeigt, daß das Vorhandensein von CD28 Autoantikörpern tendenziell mit jüngerem Alter und weiblichem Geschlecht korreliert ist.

**Tabelle 1.**
Alter und Geschlecht im Verhältnis zu anti-CD28 Autoantikör- pern

| | CD28+ | CD28- | p |
|---|---|---|---|
| Alter (±SD) [Jahre] | 52.3 ± 18.8 | 58.7 ± 19.9 | 0.073 |
| Geschlecht | | | |
| männlich | 19 | 69 | |
| weiblich | 34 | 74 | 0.146 |

SD:Standardabweichung

Die Univarianz-Analyse zeigt, dass das Auftreten von CD28 Autoantikörpern hochsignifikant mit dem atopischen Ekzem (Odds-Ratio, 25.31 [95% CI, 5.52 - 116.11]; p<0.0001), allergischem Asthma und Rhinokonjunktivitis allergica (OR 10.78 [95% CI, 5.39 - 21.55]; p<0.0001) sowie mit Autoimmunerkrankungen wie z.B. der Sklerodermie, Lupus erythematodes, Rheumatoider Arthritis, Dermatomyositis oder bullösen Autoimmunerkrankungen assoziiert ist **(Tabelle 2).** Alle anderen Krankheiten, die in dem Patientenkollektiv diagnostiziert wurden, waren nicht mit dem Auftreten von CD28 Autoantikörpern korreliert.

Um andere Einflußgrößen wie z.B. Serum-IgE auszuschließen, wurde zusätzlich eine multivariate logistische Regressionsanalyse durchgeführt. Dadurch konnte ein möglicher Einfluß des Alters, des Geschlechtes oder des IgE im Serum als Kofaktoren statistisch ausgeschlossen werden **(Tabelle 3).**

**Tabelle 2.** Assoziation zwischen Diagnose und anti-CD28 Autoantikörpern.

| | **Total** | **anti-CD28+** | **anti-CD28 -** | **Odds-Ratio** | **95% CI** | ***p**** |
|---|---|---|---|---|---|---|
| Gesunde Kontrollen | 72 | 8 | 64 | - | - | - |
| Atopische Dermatitis | 16 | 14 | 2 | 56.00 | 28.4-110.4 | *< 0:0001* |
| Allergische Rhinitis/ Asthma | 54 | 31 | 23 | 10.78 | 5.39-21.55 | *< 0.0001* |
| Autoimmunkrankheiten | 8 | 5 | 3 | 13.33 | 5.72-31.1 | *<0.01* |
| Cutanes Lymphom | 3 | 1 | 2 | 4.00 | 0.71-22.5 | *n.s.* |
| Nicht-Melanom Hautkrebs | 27 | 9 | 18 | 4.00 | 1.72-9.30 | *n.s.* |
| Geschwür am Bein | 53 | 13 | 40 | 2.60 | 1.16-5.82 | *n.s.* |
| Hautinfektionen | 24 | 5 | 19 | 2.11 | 0.76-5.82 | *n.s.* |
| Sonst. entzündl. Hauterkrankungen | 49 | 10 | 39 | 2.05 | 0.87-4.83 | *n.s.* |
| Psoriasis | 9 | 1 | 8 | 1.00 | 0.14-7.10 | *n.s.* |
| Malignes Melanom | 11 | 1 | 10 | 0.80 | 0.11-5.79 | *n.s.* |

95%-CI: 95 Prozent Konfidenz Interval; n.s.: nicht signifikant;
p*: Bonferroni-korrigierter p-Wert mit Fisher's exact test; OR: odds ratio

**Tabelle 3**. Logistische Regressionsanalyse von Faktoren, die das Vorkommen von anti-CD28 Autoantikörpern beeinflussen.

| Faktor | OR | 95% CI | *P* |
|---|---|---|---|
| Alter | 0.993 | 0.974 -1.012 | *0.443* |
| Geschlecht | 1.073 | 0.503 - 2.288 | *0.855* |
| IgE [kU/l] | 0.999 | 0.999 -1.000 | *0.077* |
| Allergische Rhinitis/ Asthma | 2.484 | 1.139 - 5.417 | *0.022* |
| Atopische Dermatitis | 62.682 | 6.296 - 624.02 | *0.0004* |
| Autoimmunkrankheiten | 8.909 | 1.572-50.48 | *0.014* |

**Beispiel 3:**

**Funktionelle Bedeutung der Autoantikörper gegen CD28**

Die funktionelle Bedeutung der Autoantikörper gegen CD28 wurde mit Hilfe einer sogenannten "gemischten Lymphozytenreaktion" (mixed lymphocyte reaction, MLR) untersucht.

Zunächst wurden Protein G-Beads (Dynal, Hamburg) nach Vorschrift mit dem CD28-Ig Fusionprotein beladen. Das Protein wurde durch Crosslinking irreversibel an die Beads gebunden, und die Bindung wurde über eine durchflußzytometrische Messung bestimmt. Anschließend wurden Serumpools hergestellt aus (1) drei Seren mit anti-CD28 Autoantikörpern von Patienten mit atopischem Ekzem (AE+), (2) zwei Seren ohne anti-CD28 Autoantikörper von Patienten mit atopischem Ekzem (AEØ), (3) zwei Seren mit anti-CD28 Autoantikörpern von Patienten ohne atopisches Ekzem (G+) und (4) aus sieben Seren ohne anti-CD28 Autoantikörper von Patienten ohne atopisches Ekzem (GØ).

Die Poolseren wurden nacheinander über die Beads aufgereinigt, so daß man die Fraktionen mit den Antikörpern gegen CD28 und gegen humanes Fc erhielt. Die Eluate wurden anschließend im Western Blot auf das Vorhandensein von Antikörpern gegen CD28 getestet.

Anschließend wurde mit den Eluaten und den Poolseren ein Proliferationstest im Rahmen einer MLR durchgeführt. Die Zellinien wurden in RPMI 1640 Medium + 10% Fötales Kälberserum (FKS) gezüchtet. Für die MLR wurden Jurkat Zellen ($10^4$ Zellen/ml) zusammen mit bestrahlten (Dosis: 30 Gy) Raji Zellen ($10^4$/ml) kultiviert. Die Eluate (50 $\mu$l) wurden in verschiedenen Ansätzen hinzugegeben. In einigen Ansätzen wurden 4 $\mu$g CTLA-4-Ig (R&D Systems, Minneapolis, USA) zur Induktion einer Anergie zugesetzt. Nach 2 Tagen wurde den Kulturen 5-bromo-2'-deoxyuridin (BrdU) zugegeben und für 5 Stunden inkubiert (Neuber et al., Immunology 2003, 109: 24-31). Anschließend konnte die Proliferation der Zellen anhand einer Farbreaktion gemessen werden.

Die Experimente zeigten **(Fig. 3)**, daß die Eluate von Seren mit Autoantikörpern gegen CD28 die Zellproliferation stark stimulierten, während die Seren ohne Autoantikörper die Proliferation hemmten.

Die Stimulation des CTLA-4 Rezeptors auf T-Lymphozyten versetzt die Zellen durch eine Hemmung der stimulatorischen Signale in einen anergen Zustand (Sharpe et al, Nature Rev 2002, 2: 116-126). Deshalb wurde das CTLA-4-Ig Fusionsprotein als Kontrolle in den Proliferationsexperimenten eingesetzt (Linsley et al., J Exp Med 1991, 174: 561-569). Es zeigte sich, dass Seren mit Autoantikörpern gegen CD28 den anergen Zustand durchbrechen können und die T-Zellen erneut proliferieren.

**Beispiel 4:**

**Herstellung eines CD28-GST-Fusionsproteins**

**RNA-Präparation aus humanem Vollblut**

Zunächst wurde aus humanem Vollblut mit Hilfe des *QIAamp RNA Blood Mini*-Kit der der Firma Qiagen (Hilden, Katalognummer: 52304) RNA präpariert.

**RT-PCR zur Herstellung von cDNA**

5 $\mu$g der präparierten RNA wurden mit dem *superscript*-Kit (Invitrogen, Karlsruhe) mit *random hexamer*-Primern (Katalognummer: 53034) in Blut-cDNA umgeschrieben.

**Klonierung der CD28 cDNA**

Anschließend wurde der für den extrazellulären Bereich (IgGartige Domäne, ohne Transmembranregion und Signalpeptid) dieses Proteins kodierende cDNA-Bereich mit Hilfe einer Polymerasekettenreaktion (PCR) amplifiziert. Als Referenz diente die *online* bei NCBI abfragbare Sequenz NM_006139 (SEQ ID NO: 3).

Die amplifizierte cDNA kodiert für die Aminosäuresequenz:

PSIQVTGNKILVKQSPMLVAYDNAVNLSCKYSYNLFSREFRASLHKGLDSAVEVCVVYGNY-SQQLQVYSKTGFNCDGKLGNESVTFYLQNLYVNQTDIYFCKIEVMYPPPYLDNEKSNGTI-IHVKGKHLCPSPLF (SEQ ID NO: 2)

Als Primer dienten:

Sense 5'-AAAGAATTCCCTTCAATTCAAGTAACAGGAAAC-3' (SEQ ID NO: 4)
Antisense 5'-AAACCCGGGAAATAGGGGACTTGGACAAAG-3' (SEQ ID NO: 5)

Die Integration der cDNA in die *multiple-cloning-site* des Vektors pGEX-4T-1 (Amersham Biosciences, Freiburg, Katalognummer: 27-4580-01) erfolgte über Schnittstellen für die Restriktionsenzyme EcoRI und SmaI, die bereits in die zur Amplifikation eingesetzten Primer 5' integriert waren (in der Primersequenz unterstrichen). Dazu wurde das PCR-Amplifikat ebenso wie der Vektor pGEX-4T-1 zunächst mit SmaI (New England Biolabs, Frankfurt a.M., Katalognummer: #R0141S) und anschließend mit EcoRI (New England Biolabs, Katalognummer #R0101S) im Reaktionspuffer NEB4 bei 20°C bzw. 37°C für jeweils zwei Stunden inkubiert. Anschließend erfolgte eine Aufreinigung der geschnittenen DNA über das *Agarose Gel DNA Extraction-Kit* der Firma Roche (Basel, Katalognummer: 1696505). Der linearisierte Vektor wurde anschließend dephosphoryliert, wozu die eluierte DNA bei 37°C mit 10 U alkalischer Phosphatase (Roche, Katalognummer: 713023) nach Zugabe des entsprechenden Puffers inkubiert wurde. Die Restriktionsansätze wurden anschließend in einem einprozentigen Agarose-Gel bei 100 V aufgetrennt. Nach Ethidium-Bromid-Färbung konnte eine Bande von ca. 400 bp auf einem UV-Transilluminator visualisiert und ausgeschnitten werden. Die geschnittene cDNA wurde dann mit Hilfe des Roche Agarose-gel-Elution kits in 50 µl $H_2O$ eluiert. Anschließend wurde ein Ligationsansatz mit T4-Ligase (Invitrogen, Katalognummer: E111-01), 100 ng Vektor und 200 ng cDNA-Fragment angesetzt und bei 12°C mehrere Stunden inkubiert. Hieraus ergibt sich ein Konstrukt, bei dem die Glutathion S-Transferase 5'-wärts des im Leserahmen befindlichen CD28-Bereichs liegt.

Anschließend wurden kompetente Bakterien (XL1 Blue, HB 101) mit einem Fünftel des Ligationsansatzes transformiert. Dazu wurde zu 50 µl auf Eis aufgetauter kompetenter Bakterien ein Fünftel eines Ligationsansatzes oder 0,5 µg einer DNS-Präparation hinzupipettiert und 30 min auf Eis inkubiert.

Anschließend wurden die Bakterienansätze 5 min auf 37°C erwärmt. Danach wurden 950 µl SOC Medium zugegeben und zwischen 50 µl und 1 ml des Ansatzes auf LB-Agar-Platten mit 150 µg/ml Ampicillin mit einer Pipette gleichmäßig ausgestrichen und über Nacht bei 37°C inkubiert.

**Minipräparation von Plasmid-DNA aus Bakterien**

Einzelne Kolonien transformierter Bakterien wurden in 2,5 ml Medium mit entsprechendem Antibiotikumszusatz angeimpft und über Nacht inkubiert. Die Bakterien wurden dann bei 2400 g pelletiert, der Überstand abgesaugt und das Pellet in 200 µl STET (8% Sukrose, 5% Triton X-100, 50 mM Tris-HCl, pH 8.0, 50 mM EDTA) mit 1 µg/ml Lysozym auf dem Schüttler resuspendiert. Anschließend wurde der Ansatz für 2 Minuten auf 95°C erhitzt und dann 10 min bei 16000g zentrifugiert. Das Sediment wurde nun mit einem Zahnstocher entfernt und verworfen. Dem Überstand wurden 10 µl 5%iger CTAB-Lösung (Cetyl-Trimethyl-Ammoniumbromid) zugegeben, kurz geschüttelt und das Präzipitat bei 16000 g pelletiert. Der Überstand wurde abgesaugt und das Pellet in 300 µl 1,2 M NaCl auf dem Schüttler gelöst. Dann wurden 750 µl Ethanol 100% zugegeben, der Ansatz kräftig geschüttelt und bei 10 min bei 16000g zentrifugiert. Der Überstand wurde abgesaugt, das Pellet nun in 1 ml 70%igem Ethanol gewaschen, 5 min bei 16000 g zentrifugiert und der Überstand erneut verworfen. Das DNA-Pellet wurde nun getrocknet und in 30 µl H2O aufgenommen.

Die Richtigkeit der Sequenz des eingefügten Bereichs erfolgte durch anschließende Sequenzierung.

**Präparation des rekombinanten CD28-GST-Fusionsproteins**

Kompetente BL21-RILsuppl. Bakterien (ein proteasedefizienter E. coli-Stamm) wurden mit dem cDNA-Konstrukt transformiert und nach halbstündiger Vorinkubation in SOC-Medium bei 37°C auf LB-Agar-Platten mit 150 μg/ml Ampicillin ausgestrichen. Nach 14stündiger Inkubation wurde eine 30 ml LB-Medium mit Ampicillin mit einer Kolonie beimpft.

Diese Vorkultur wurde über Nacht bei 37°C im Schüttler inkubiert. Diese wurde dann in 500 ml LB-Medium mit Ampicillin überführt und bis zu einer optischen Dichte von 0,6-0,8 bei 600 nm unter ständigem Schütteln bei 37°C inkubiert (ca. 1,5 Stunden).

Sobald diese Dichte erreicht war, wurde die Proteinexpression mit 1 mM IPTG (Isopropyl bD-thiogalactopyranosid, Biomol, Hamburg, Katalognummer: 05684-1) induziert. Nach ca. 4 Stunden wurden die Bakterien bei 4000g und 4°C für 10 Minuten pelletiert, der Kulturüberstand verworfen und das Pellet in 5-10 ml eiskaltem PBS resuspendiert. Diese Suspension wurde nun fünfmal einer Ultraschallbehandlung von je 10 Sekunden Dauer unterzogen (Branson Sonifier 250, Stufe 6) und anschließend 20 Minuten bei 30000g zentrifugiert. Nun erfolgte die Affinitätsreinigung über den GST-Tag. Glutathion-Sepharose 4B (Amersham Biosciences, Katalognummer: 27-4574-01) wurde bis zu einem Bettvolumen von 1 ml in eine gravitationsgetriebene Polypropylensäule geladen und in einem fünffachem Volumen PBS äquilibriert.

Anschließend wurde das Bakterienlysat aufgeladen und der Durchfluss erneut auf die Säule gegeben. Der zweite Durchfluss wurde verworfen und die Säule mit dem fünf- bis zehnfachen des Bettvolumens PBS gewaschen. Eluiert wurde in 10 mM reduziertem Glutathion, 50 mM Tris pH 7,5, 100 mM NaCl, 10% Glycerol. Das eluierte Protein wurde anschließend aliquotiert und bei - 80 °C aufbewahrt.

**Benötigte Materialien**

| | |
|---|---|
| PBS | 137 mM NaCl<br>2,7 mM KCl<br>7 , 4 mM $Na_2HPO_4$<br>1,5 mM $KH_2PO_4$ |
| STET | 8% Sucrose<br>0,1% Triton X<br>50 mM EDTA<br>50 mM Tris pH 8 |
| TAEx50 | 2 M Tris Base<br>5,71% Eisessig<br>50 mM EDTA |
| TE | 20 mM Tris pH 7,5<br>1 mM EDTA |
| Auftragpuffer x 5 (für Nukleinsäuren) | 40% Sucrose<br>0,25% Bromphenolblau<br>0,25% Xylene X<br>in TE |
| SOC Medium | 2% bacto-trypton<br>0,5% Bacto Hefe Extrakt<br>10 mM NaCl<br>2,5 mM KCl<br>5 N NaOH auf pH 7,0 |
| Autoklavieren und anschließend auf 60°C abkühlen. | |
| | 10 mM $MgCl_2$<br>10 mM Glukose |

(fortgesetzt)

| | |
|---|---|
| Dyt Medium | 1,6% Bacto-tryptone<br>1% Bacto Hefe Extrakt<br>100 mM Nacl |
| LB Medium | 1% Bacto-tryptone<br>0,5% Bacto Hefe Extrakt<br>200 mM NaCl |
| mit NaOH auf pH 7,5 einstellen | |
| Y-broth | LB Medium<br>4 mM $MgSO_4$<br>5 mM KCl |
| TFB 1 | 15% Glycerin<br>10 mM $CaCl_2$<br>30 mM Kaliumacetat |
| mit Essigsäure auf pH 5,8 einstellen | |
| | 100 mM RbCl |
| | 50 mM $MnCl_2$ |
| TFB 2 | 15% Glycerin<br>10 mM MOPS<br>75 mM $CaCl_2$<br>10 mM RbCl |
| LB Medium | 1% Bacto-tryptone<br>0,5% Bacto Hefe Extrakt<br>200 mM NaCl |
| mit NaOH auf pH 7,5 einstellen | |

**Herstellung kompetenter Bakterien**

XL1blue Bakterien wurden auf einer LB-Platte ausgestrichen und über Nacht bei 37°C inkubiert. Morgens wurden einige Kolonien mit je 2 ml Y-broth angeimpft und 2 Stunden bei 37°C im Schüttler inkubiert. Anschließend wurden diese Vorkulturen in 500 ml Y-broth gegossen und bis zu einer OD bei 600 nm von 0,3-0,35 inkubiert. Dann wurde die Kultur auf zwei 50 ml Polypropylenröhrchen verteilt, kurz auf Eis gestellt und bei 4°C und 2000g pelletiert. Der Überstand wurde dekantiert, das Pellet in je 15 ml in TFB 1 (15% Glycerin, 10 mM $CaCl_2$, 30 mM Kaliumacetat, mit Essigsäure auf pH 5,8 eingestellt, 100 mM RbCl, 50 mM $MnCl_2$) resuspendiert und 60-90 Minuten auf Eis gestellt.

Anschließend wurde erneut mit 2000g pelletiert, der Überstand dekantiert und das Pellet in je 2 ml TFB 2 (15% Glycerin, 10 mM MOPS, 75 mM $CaCl_2$, 10 mM RbCl) resuspendiert. Die Bakterien wurden dann in 200 µl Aliquots aufgeteilt und sofort in Flüssigstickstoff schockgefroren und anschließend bei -80°C gelagert.

**Beispiel 5:**

**ELISA zum Nachweis spezifischer Antikörper gegen die extrazelluläre Domäne des humanen CD28**

Die Kavitäten von Mikrotiterplatten (Maxisorp, Nunc) werden mit 100µl monoklonalem Maus anti-Gluthation-S-Transferase (GST) Antikörper (spezifisch für GST aus Schistosoma japonicum, 1:2000 in PBS vorverdünnt) beschichtet. Nach einer Inkubationszeit von 1 Stunde bei Raumtemperatur werden die Kavitäten dreimal mit PBS + 0,1% Tween 20 gewaschen.

Dann wird mit 250µl PBS mit 1% Magermilchpulver und 0,1% Tween 20 für 1 Stunde bei Raumtemperatur blockiert. Anschließend wird erneut dreimal mit PBS + 0,1% Tween 20 gewaschen.

Während in die Vertiefungen der Spalten 1 und 2 jeweils 100µl 1:2 vorverdünntes CD28-GST Antigen (PBS + 0,1% Tween 20) pipettiert wurden, kommen in die Vertiefungen der dritten und vierten Spalte nur je 100µl PBS + 0,1 % Tween 20. Entsprechend werden die übrigen Spalten der Mikrotiterplatte vorbereitet.

Nach einer Stunde bei Raumtemperatur folgen erneut drei Waschschritte. Nun werden die Patientenseren sowie das positive Kontrollserum, ein polyklonaler Kaninchen anti-human CD28 Antikörper (Santa Cruz, Heidelberg), Konzentration 1µg/ml, 1:200 in PBS + 0,1 % Tween 20 vorverdünnt.

Der Leerwert enthielt das Antigen, aber kein Kontrollserum, die Negativkontrolle enthielt das Kontrollserum, aber kein Antigen und der Blank enthielt weder Kontrollserum noch Antigen. Jedes Patientenserum wurde in Doppelbestimmungen gegen Antigen und - zum Ausschluss unspezifischer Reaktionen - gegen PBS + 0,1 % /Tween 20 gemessen.

Anschließend wurde die Mikrotiterplatte für 1 h bei Raumtemperatur inkubiert und anschließend dreimal gewaschen. Im nächsten Schritt wurde der Sekundärantikörper hinzugegeben. In die Vertiefungen mit dem Kontrollserum (B 1-4) wurden je 100µl eines anti Kaninchen IgG Antikörpers (Fc-spezifisch; Sigma, München) und für die Patientenseren je 100µl eines anti-human IgG Antikörpers (Fc-spezifisch; Sigma, München) verwendet. Beide Antikörper wurden 1: 5000 vorverdünnt und sind mit alkalischer Phosphatase markiert. Die Inkubationszeit betrug bei Raumtemperatur 60 Minuten.

Danach wurde fünfmal gründlich gewaschen und in jede Vertiefung wurden 100µl der p-Nitrophenyl Substratlösung gegeben (pNPP Substrat Tabletten Set, Sigma, München) und sechzig Minuten bei Raumtemperatur im Dunkeln inkubiert. Die Farbentwicklung wurde nach 30 und nach 60 Minuten bei 405 nm gemessen.

Das Ergebnis für jedes Serum wurde nach der folgenden Formel berechnet:

$$\text{Anti-CD28} = \frac{OD_{Serum}\ [60\ \text{min}] - OD_{LW}}{OD_{LW}}$$

Der Quotient wurde für alle getesteten Seren errechnet. Der Grenzwert wurde mit den 72 Seren der gesunden Probanden errechnet. Es zeigte sich, daß 95 % aller errechneten Quotienten der gesunden Probanden unter 9 lagen, so daß Werte, die >10 waren als positiv gewertet wurden, d.h. sie enthielten Autoantikörper gegen CD28.

Zusätzlich wurden Seren, die sicher keine Autoantikörper gegen CD28 enthielten und solche, die sicher CD28 Autoantikörper enthielten in einer Verdünnungsreihe getestet. Es zeigte sich, dass Seren, die CD28 Autoantikörper enthalten, auch noch bei einer Verdünnung von 1 : 700 eine eindeutig höhere OD zeigten als die negativen Seren **(Fig. 4).**

Kreuzreagierende Antikörper in den Patientenseren gegen GST konnten ausgeschlossen werden. Dazu wurden 32 Seren getestet und in keinem Serum fanden sich Antikörper gegen das in diesem ELISA verwendete GST.

Bei allen Patienten mit einer atopischen Erkrankung lagen die IgE-Werte im Serum vor. Die Konzentration an IgE ist ein Parameter für den Schweregrad einer atopischen Erkrankung. Die Titer für Autoantikörper gegen CD28 korrelieren bei diesen Patienten signifikant mit der Höhe des Serum IgE-Titers. Daraus kann man schließen, dass die Höhe der anti-CD28 Autoantikörper Titer ebenfalls mit der Schwere der Atopie korrelieren.

**Abbildungen:**

**Fig. 1:**

Spaltprodukte nach enzymatischem Verdau des CD28-Ig Fusionsproteins mit Trypsin.

Spalte 1: CD28 markiert mit Maus anti-human CD28 moAk (monoklonalen Antikörper). Spalte 2: CD28 markiert mit einem biotinylierten polyklonalen Maus anti-human CD28 Ak. Einige kleinere Fragmente des CD28-Ig Fusionsproteins werden mit diesem polyklonalen Ak nachgewiesen, nicht aber mit dem monoklonalen Antikörper. Spalten 3-5: Nachweis von Spaltprodukten des Ig-Anteils mit dem Kaninchen anti-human IgG-Ak (Spalte 3) sowie mit dem Ziege anti-human IgG Ak (Spalte 4) und mit dem Maus anti-human Fc Ak (Spalte 5).

**Fig. 2:**

Immunoblots von 4 Patienten mit atopischem Ekzem (AD) und Autoantikörpern gegen CD28 sowie einem Patient ohne Autoantikörper. Als Beispiel für CD28 Antikörper bei Autoimmunerkrankungen (AI) wird das Ergebnis mit dem Serum eines Patienten mit Sklerodermie gezeigt. Bei den hier gezeigten Patienten mit Epidermolysis bullosa acquisita (AI) and Psoriasis vulgaris (PS) konnten keine Autoantikörper gegen CD28 nachgewiesen werden.

**Fig. 3:**

MLR mit betrahlten Raji Zellen and vitalen Jurkat Zellen. Die Proliferation wurde mit dem Einbau von BrdU nach 2 Kulturtagen gemessen. Ergebnisse werden als %-Stimulation der spontanen Proliferation (Kontrolle (control)) dargestellt. CTLA4-Ig hemmt die Proliferation, während Eluate mit CD28 Autoantikörpern (CD28 auto-Ab) die T-Zell-Proliferation signifikant stimulieren. Kostimulation mit CTLA4-Ig und Autoantikörpern gegen CD28 zeigte eine signifikant verminderte Hemmung der Jurkat Zell Proliferation. ** $P < 0.01$, signifikant im Vergleich zur Kontrolle.

**Fig. 4:**

Untersuchung von Seren auf anti-CD28 Autoantikörper im ELISA. Die OD der Seren, die sicher anti-CD28 Autoantikörper enthalten, liegt auch noch bei hohen Verdünnungen noch deutlich höher als bei eindeutig anti-CD28 Autoantikörper-negativen Seren.

**Fig. 5:**

Darstellung der Korrelation zwischen anti-CD28 Autoantikörpern und Serum-IgE bei Patienten mit Atopie bzw. atopischem Ekzem. Der Korrelationekoeffizient beträgt 0.206 und das Signifikanznieveau 0.012.

## Patentansprüche

1. Verwendung von CD28 zur in vitro Diagnose allergischer Erkrankungen, atopischer Erkrankungen und/oder Autoimmunerkrankungen, wobei CD28 ein CD28-Molekül ganzer Länge oder ein extrazelluläres Fragment davon umfaßt.

2. Verwendung nach Anspruch 1, **dadurch gekennzeichnet, daß** man eine Probe von einem Patienten auf das Vorhandensein von anti-CD28 Autoantikörpern untersucht, indem man die Probe mit CD28 in Kontakt bringt, wobei eine Bindung der Autoantikörper an CD28 auf das Vorliegen einer allergischen Erkrankung, atopischen Erkrankung und/oder Autoimmunerkrankung hinweist.

3. Vefahren zur Diagnose allergischer Erkrankungen, atopischer Erkrankungen und/oder Autoimmunerkrankungen, **dadurch gekennzeichnet, daß** man eine Probe eines Patienten auf das Vorhandensein von anti-CD28 Autoantikörpern untersucht, indem man die Probe mit CD28 in Kontakt bringt, wobei eine Bindung der Autoantikörper an CD28 auf das Vorliegen einer allergischen Erkrankung, atopischen Erkrankung und/oder Autoimmunerkrankung hinweist, wobei CD28 ein CD28-Molekül ganzer Länge oder ein extrazelluläres Fragment davon umfaßt.

4. Verfahren oder Verwendung nach den Ansprüchen 1 bis 3, **dadurch gekennzeichnet, daß** das extrazelluläre Fragment die Sequenz gemäß SEQ ID NO: 2 aufweist.

5. Verfahren oder Verwendung nach den Ansprüchen 1 bis 4, **dadurch gekennzeichnet, daß** das CD28-Molekül ganzer Länge oder das extrazelluläre Fragment davon Teil eines Fusionsproteins sind.

6. Verfahren oder Verwendung nach Anspruch 5, **dadurch gekennzeichnet, daß** das Fusionsprotein ferner Glutathion-S-Transferase umfaßt.

7. Verfahren oder Verwendung nach den Ansprüchen 2 bis 6, **dadurch gekennzeichnet, daß** die Probe eine Blutprobe oder Serumprobe ist.

8. Verfahren oder Verwendung nach den Ansprüchen 1 bis 7, **dadurch gekennzeichnet, daß** CD28 an einen Träger gebunden ist.

9. Verfahren oder Verwendung nach Anspruch 8, **dadurch gekennzeichnet, daß** man die Bindung von anti-CD28 Autoantikörpern an CD28 untersucht, indem man den Träger mit markierten anti-Immunglobulin-Antikörpern gegen Antikörper der Spezies, der der Patient angehört, kontaktiert und die markierten Antikörper nachweist.

10. Verfahren oder Verwendung nach Anspruch 9, **dadurch gekennzeichnet, daß** der Patient ein Mensch ist und die anti-Immunglobulin-Antikörper anti-humane Immunglobulin-Antikörper sind.

11. Verfahren oder Verwendung nach den Ansprüchen 9 und 10, **dadurch gekennzeichnet, daß** die anti-Immunglobulin-Antikörper mit einem Enzym, Biotin, einem radioaktiven Isotop oder einem Fluoreszenzfarbstoff markiert sind.

12. Verfahren oder Verwendung nach den Ansprüchen 9 bis 11, **dadurch gekennzeichnet, daß** man die Untersuchung in einem Blot, einem ELISA, einem RIA, einer FACS-Untersuchung oder in einem Flüssigphasen-Detektionssystem durchführt.

13. Verfahren oder Verwendung nach den Ansprüchen 1 bis 12, **dadurch gekennzeichnet, daß** die allergische Erkrankung eine Rhinokonjunktivitis allergica oder allergisches Asthma bronchiale ist.

14. Verfahren oder Verwendung nach den Ansprüchen 1 bis 12, **dadurch gekennzeichnet, daß** die atopische Erkrankung eine atopische Dermatitis ist.

15. Verfahren oder Verwendung nach den Ansprüchen 1 bis 12, **dadurch gekennzeichnet, daß** die Autoimmunerkrankung Sklerodermie, Lupus erythematodes, Rheumatoide Arthritis, Dermatomyositis oder eine bullöse Autoimmunerkrankung ist.

16. Verfahren oder Verwendung nach den Ansprüchen 1 bis 14, **dadurch gekennzeichnet, daß** man ferner die Konzentration an Gesamt-IgE in der Probe bestimmt, wobei eine Konzentration von 100 IU/ml Gesamt-IgE oder mehr auf das Vorliegen einer allergischen Erkrankung und/oder atopischen Erkrankung hinweist.

17. Verfahren oder Verwendung nach den Ansprüchen 1 bis 16, **dadurch gekennzeichnet, daß** eine hohe Konzentration von Autoantikörpern gegen CD28 auf ein besonderes Risikos, eine besonders schwere Erkrankung oder eine besondere Schwere der Erkrankung hinweist.

18. Kit zur Diagnose allergischer Erkrankungen und/oder atopischer Erkrankungen, **dadurch gekennzeichnet, daß** es CD28 und markierte anti-Immunglobulin Antikörper umfaßt, wobei CD28 ein CD28-Molekül ganzer Länge oder ein extrazelluläres Fragment davon umfaßt.

19. Kit nach Anspruch 18, **dadurch gekennzeichnet, daß** das extrazelluläre Fragment die Sequenz gemäß SEQ ID NO: 2 aufweist.

20. Kit nach den Ansprüchen 18 und 19, **dadurch gekennzeichnet, daß** das CD28-Molekül ganzer Länge oder das extrazelluläre Fragment davon Teil eines Fusionsproteins sind.

21. Kit nach Anspruch 20, **dadurch gekennzeichnet, daß** das Fusionsprotein ferner Glutathion-S-Transferase umfaßt.

22. Kit nach den Ansprüchen 18 bis 21, **dadurch gekennzeichnet, daß** es ferner markierte anti-IgE Antikörper umfaßt.

23. Kit nach den Ansprüchen 18 bis 21, **dadurch gekennzeichnet, daß** die markierten Antikörper mit einem Enzym, Biotin, einem radioaktiven Isotop oder einem Fluoreszenzfarbstoff markiert sind.

## Claims

1. Use of CD28 for the in vitro diagnosis of allergic diseases, atopic diseases and/or autoimmune diseases, wherein CD28 comprises a full length CD28 molecule or an extracellular fragment thereof.

2. The use of claim 1, **characterized in that** a sample from a patient is analyzed for the presence of anti-CD28 autoantibodies by contacting the sample with CD28, **characterized in that** binding of the autoantibodies to CD28 indicates the presence of an allergic disease, atopic disease and/or autoimmune disease.

3. Method for the diagnosis of allergic diseases, atopic diseases and/or autoimmune diseases, **characterized in that** a sample from a patient is analyzed for the presence of anti-CD28 autoantibodies by contacting the sample with CD28, **characterized in that** binding of the autoantibodies to CD28 indicates the presence of an allergic disease, atopic disease and/or autoimmune disease, wherein CD28 comprises a full length CD28 molecule or an extracellular fragment thereof.

4. Method or use of claims 1 to 3, **characterized in that** the extracellular fragment has the sequence according to SEQ ID NO: 2.

5. Method or use of claims 1 to 4, **characterized in that** the full length CD28 molecule or the extracellular fragment thereof are part of a fusion protein.

6. Method or use of claim 5, **characterized in that** the fusion protein further comprises glutathion-S-transferase.

7. Method or use of claims 2 to 6, **characterized in that** the sample is a blood sample or serum sample.

8. Method or use of claims 1 to 7, **characterized in that** CD28 is bound to a support.

9. Method or use of claim 8, **characterized in that** binding of anti-28 autoantibodies to CD28 is analyzed by contacting the support with labeled anti-immunoglobulin antibodies to antibodies of the species to which the patient belongs, and by detecting the labeled antibodies.

10. Method or use of claim 9, **characterized in that** the patient is human and the anti-immunoglobulin antibodies are anti-human immunoglobulin antibodies.

11. Method or use-of claims 9 and 10, **characterized in that** the anti-immunoglobulin antibodies are labeled with an enzyme, biotin, a radioactive isotope or a fluorescent dye.

12. Method or use of claims 9 to 11, **characterized in that** the analysis is carried out in a blot, an ELISA, an RIA, an FACS analysis or a liquid phase detection system.

13. Method or use of claims 1 to 12, **characterized in that** the allergic disease is rhinoconjunctivitis allergica or allergic asthma bronchiale.

14. Method or use of claims 1 to 12, **characterized in that** the atopic disease is an atopic dermatitis.

15. Method or use of claims 1 to 12, **characterized in that** the autoimmune disease is sclerodermia, lupus erythematodes, rheumatoid arthritis, dermatomyositis or a bullous autoimmune disease.

16. Method or use of claims 1 to 14, **characterized in that**, furthermore, the concentration of total IgE in the sample is determined, wherein a concentration of 100 IU/ml total IgE or more indicates presence of an allergic disease, and/or atopic disease.

17. Method or use of claims 1 to 16, **characterized in that** a high concentration of autoantibodies to CD28 indicates a special risk, an especially severe disease or an especially intense disease.

18. A kit for the diagnosis of allergic diseases and/or atopic diseases, **characterized in that** it comprises CD28 and labeled anti-immunoglobulin antibodies, wherein CD28 comprises a full length CD28 molecule or an extracellular fragment thereof.

19. Kit of claim 18, **characterized in that** the extracellular fragment has the sequence according to SEQ ID NO: 2.

20. Kit of claims 18 and 19, **characterized in that** the full length CD28 molecule or the extracellular fragment thereof are part of a fusion protein.

21. Kit of claim 20, **characterized in that** the fusion protein further comprises glutathion-S-transferase.

22. Kit of claims 18 to 21, further comprising labeled anti-IgE antibodies.

23. Kit of claims 18 to 22, **characterized in that** the labeled antibodies are labeled with an enzyme, biotin, a radioactive isotope or a fluorescent dye.

**Revendications**

1. Utilisation de CD28 pour le diagnostic in vitro de maladies allergiques, de maladies atopiques et/ou de maladies auto-immunes, dans laquelle CD28 comprend une molécule de CD28 complète ou un fragment extracellulaire de celle-ci.

2. Utilisation selon la revendication 1, **caractérisée en ce qu'**on examine un échantillon d'un patient quant à la présence d'auto-anticorps anti-CD28 en mettant en contact l'échantillon avec CD28, une liaison des auto-anticorps à CD28 indiquant la présence d'une maladie allergique, d'une maladie atopique et/ou d'une maladie auto-immune.

3. Procédé pour le diagnostic de maladies allergiques, de maladies atopiques et/ou de maladies auto-immunes, **caractérisé en ce qu'**on examine un échantillon d'un patient quant à la présence d'auto-anticorps anti-CD28 en mettant en contact l'échantillon avec CD28, de sorte qu'une liaison des auto-anticorps à CD28 indique la présence d'une maladie allergique, d'une maladie atopique et/ou d'une maladie auto-immune, CD28 comprenant une molécule de CD28 complète ou un fragment extracellulaire de celle-ci.

4. Procédé ou utilisation selon les revendications 1 à 3, **caractérisés en ce que** le fragment extracellulaire présente la séquence selon SEQ ID n° 2.

5. Procédé ou utilisation selon les revendications 1 à 4, **caractérisés en ce que** la molécule de CD28 complète ou le fragment extracellulaire de celle-ci sont une protéine de fusion.

6. Procédé ou utilisation selon la revendication 5, **caractérisés en ce que** la protéine de fusion comprend en outre de la glutathion-S-transférase.

7. Procédé ou utilisation selon les revendications 2 à 6, **caractérisés en ce que** l'échantillon est un échantillon de sang ou un échantillon de sérum.

8. Procédé ou utilisation selon les revendications 1 à 7, **caractérisés en ce que** CD28 est fixée sur un support.

9. Procédé ou utilisation selon la revendication 8, **caractérisés en ce qu'**on examine la liaison d'auto-anticorps anti-CD28 à CD28 en mettant le support en contact avec des anticorps anti-immunoglobuline marqués qui sont dirigés contre des anticorps de l'espèce à laquelle appartient le patient, et on détecte les anticorps marqués.

10. Procédé ou utilisation selon la revendication 9, **caractérisés en ce que** le patient est un sujet humain et les anticorps anti-immunoglobuline sont des anticorps anti-immunoglobuline humaine.

11. Procédé ou utilisation selon les revendications 9 et 10, **caractérisés en ce que** les anticorps anti-immunoglobuline sont marqués avec une enzyme, la biotine, un isotope radioactif ou un colorant fluorescent.

**12.** Procédé ou utilisation selon les revendications 9 à 11, **caractérisés en ce qu'**on effectue l'analyse dans un essai sur empreintes blot, un dosage immunoenzymatique ELISA, un dosage radio-immunologique RIA, un essai de triage de cellules activé par fluorescence FACS ou dans un système de détection en phase liquide.

**13.** Procédé ou utilisation selon les revendications 1 à 12, **caractérisés en ce que** la maladie allergique est la rhinoconjonctivite allergique ou l'asthme bronchique allergique.

**14.** Procédé ou utilisation selon les revendications 1 à 12, **caractérisés en ce que** la maladie atopique est une dermatite allergique.

**15.** Procédé ou utilisation selon les revendications 1 à 12, **caractérisés en ce que** la maladie auto-immune est la sclérodermie, le lupus érythémateux, la polyarthrite rhumatoïde, la dermatomyosite ou une maladie auto-immune bulleuse.

**16.** Procédé ou utilisation selon les revendications 1 à 14, **caractérisés en ce qu'**en outre on détermine la concentration d'IgE totales dans l'échantillon, une concentration de 100 UI/ml d'IgE totales ou plus indiquant la présence d'une maladie allergique et/ou d'une maladie atopique.

**17.** Procédé ou utilisation selon les revendications 1 à 16, **caractérisés en ce qu'**une forte concentration d'auto-anticorps dirigés contre CD28 indique un risque particulier, une maladie particulièrement grave ou une particulière gravité de la maladie.

**18.** Nécessaire pour le diagnostic de maladies allergiques et/ou de maladies atopiques, **caractérisé en ce qu'**il comprend CD28 et des anticorps anti-immunoglobuline marqués, CD28 comprenant une molécule de CD28 complète ou un fragment extracellulaire de celle-ci.

**19.** Nécessaire selon la revendication 18, **caractérisé en ce que** le fragment extracellulaire présente la séquence selon SEQ ID n° 2.

**20.** Nécessaire selon les revendications 18 et 19, **caractérisés en ce que** la molécule de CD28 complète ou le fragment extracellulaire de celle-ci sont une protéine de fusion.

**21.** Nécessaire selon la revendication 20, **caractérisés en ce que** la protéine de fusion comprend en outre de la glutathion-S-transférase.

**22.** Nécessaire selon les revendications 18 à 21, **caractérisés en ce qu'**il comprend en outre des anticorps anti-IgE marqués.

**23.** Nécessaire selon les revendications 18 à 21, **caractérisés en ce que** les anticorps marqués sont marqués avec une enzyme, la biotine, un isotope radioactif ou un colorant fluorescent.

Fig. 1

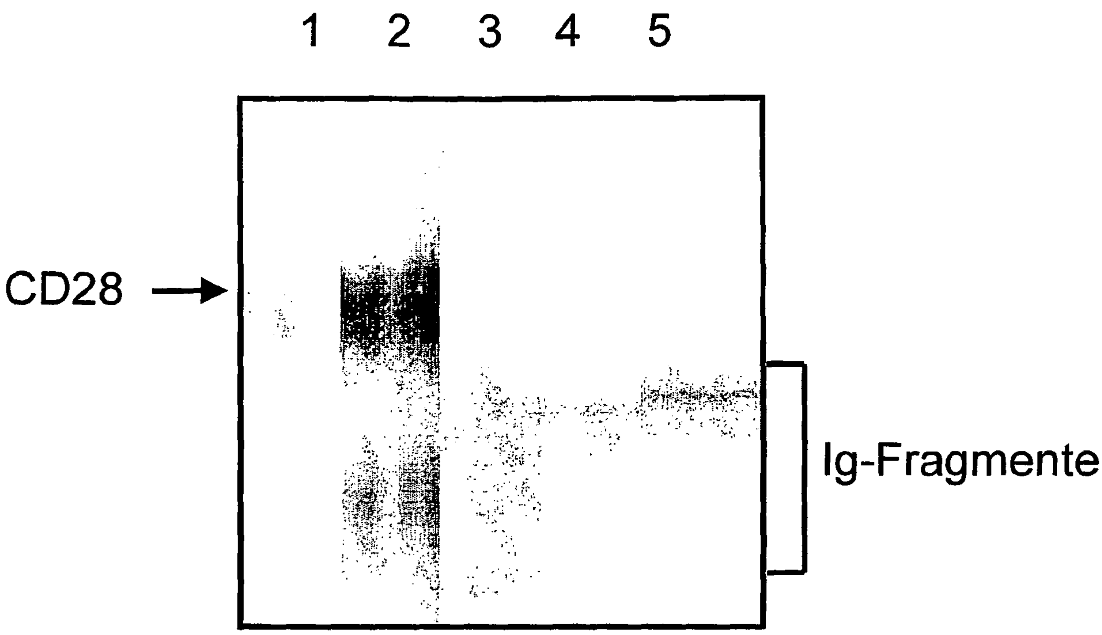


Fig. 2

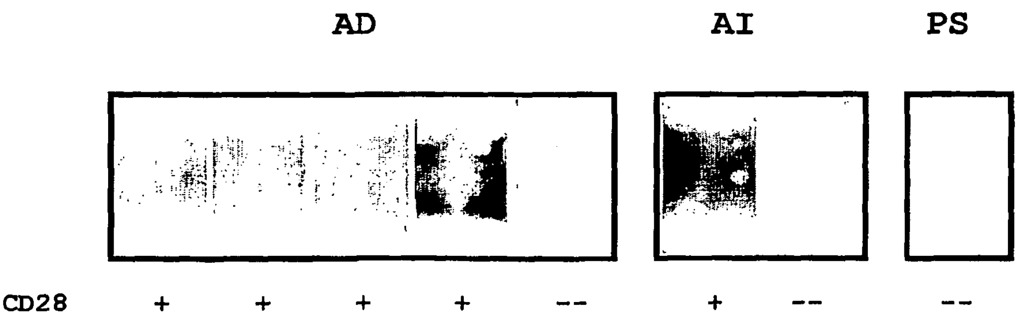

Fig. 3

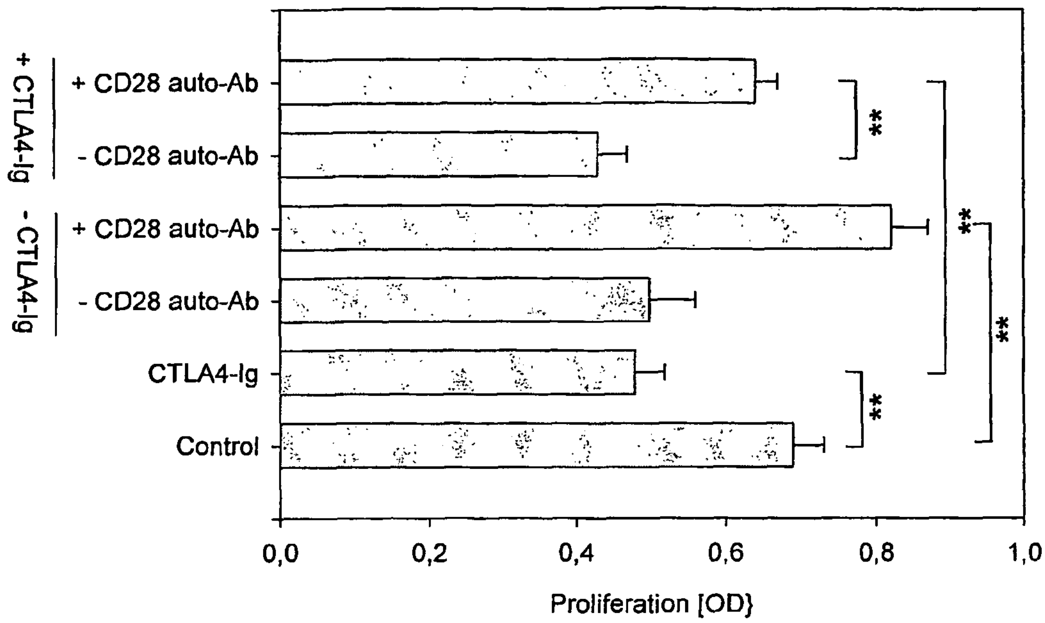
+ CTLA4-Ig
- CTLA4-Ig
+ CD28 auto-Ab
- CD28 auto-Ab
+ CD28 auto-Ab
- CD28 auto-Ab
CTLA4-Ig
Control
**
**
**
**
0,0
0,2
0,4
0,6
0,8
1,0
Proliferation [OD}

Fig. 4

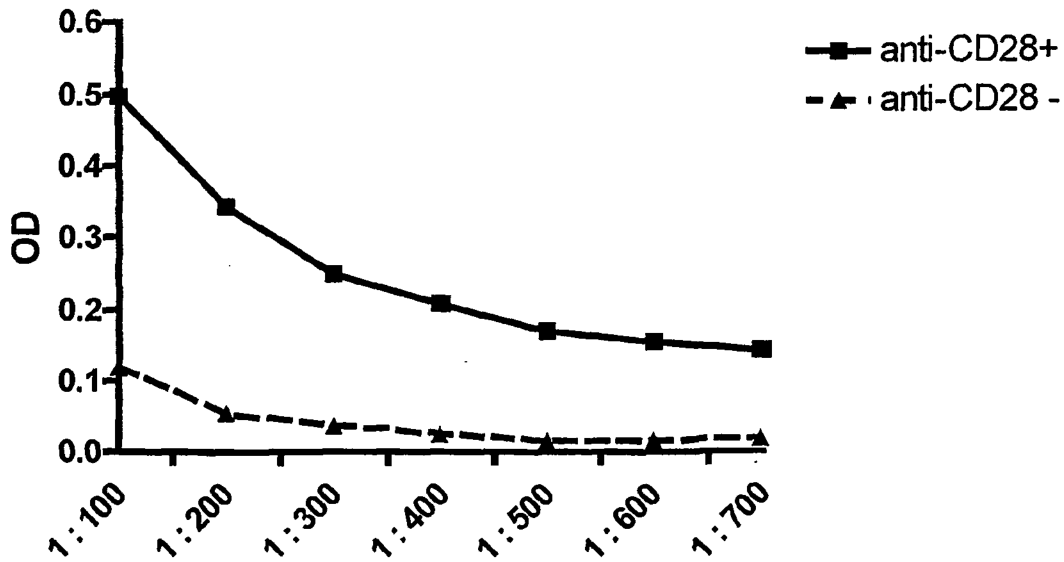
0.6
0.5
0.4
0.3
0.2
0.1
0.0
OD
1:100
1:200
1:300
1:400
1:500
1:600
1:700
anti-CD28+
anti-CD28 -

Fig. 5

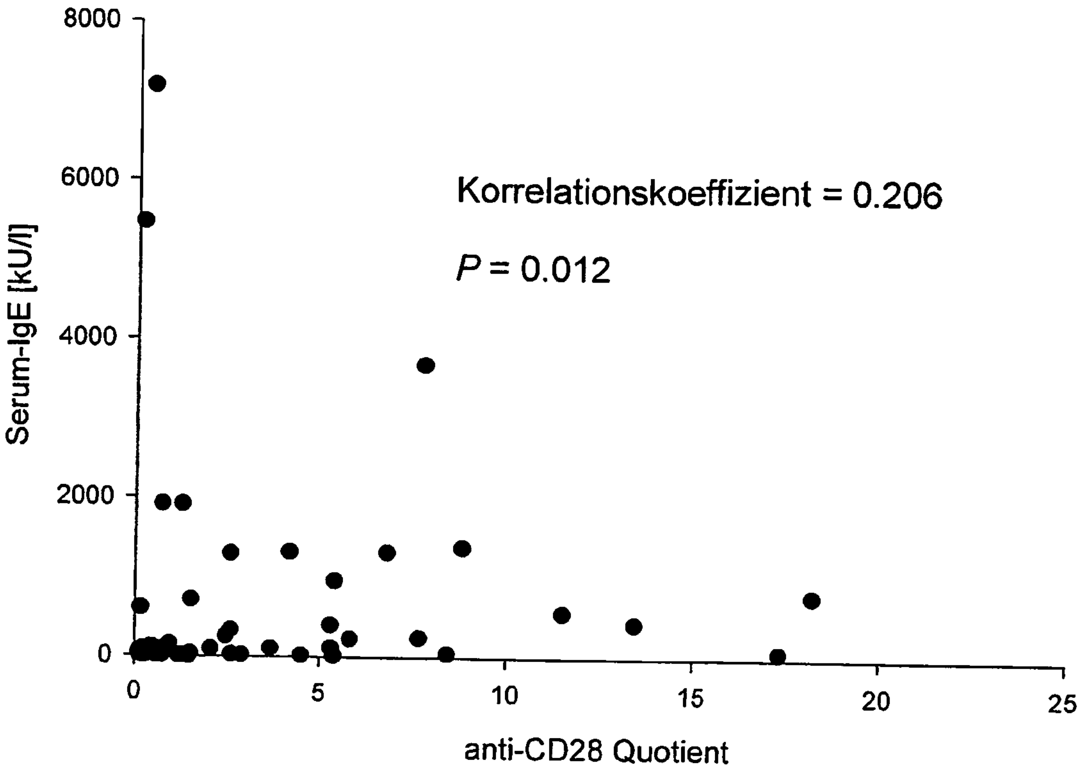
8000
6000
4000
2000
0
Serum-IgE [kU/l]
Korrelationskoeffizient = 0.206
P = 0.012
0
5
10
15
20
25
anti-CD28 Quotient

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 02066059 A
- WO 9608722 A


### In der Beschreibung aufgeführte Nicht-Patentliteratur


- **Jujo et al.** *J Allergy Clin Immunol,* 1992, vol. 90, 323-331
- **Punnonen et al.** *Proc Natl Acad Sci USA,* 1993, vol. 90, 3730-3734
- **Dreborg.** *J Am Acad Dermatol.,* 1989, vol. 21, 820-821
- **Reid et al.** *J. Allergy Clin. Immunol.,* 1986, vol. 78, 590-600
- **Varney et al.** *J. Clin. Invest.,* 1993, vol. 92, 644-651
- **Akdis ; Blaser.** *Allergy,* 2000, vol. 55, 522-530
- **Akdis et al.** *J. Clin. Invest.,* 1998, vol. 102, 98-106
- **Appleman et al.** *Immunol Rev,* 2003, vol. 192, 161-180
- **Sharpe et al.** *Nature Rev,* 2002, vol. 2, 116-126
- **Osman et al.** *Clin Rheumatol,* 1994, vol. 13, 21-27
- Lymphocytotoxic antibodies. **Swaak.** Autoantibodies. Amsterdam. Elsevier Science, 1996, 478
- **Winfield et al.** *Clin Immunol,* 1992, vol. 63, 13-16
- **Winfield et al.** *Arthritis Rheumatol,* 1975, vol. 18, 587-594
- **Morimoto et al.** *J Clin Invest,* 1987, vol. 79, 762-768
- **Tanaka et al.** *Arthritis Rheum,* 1989, vol. 32, 398-405
- **Sakane et al.** *J Clin Invest,* 1979, vol. 63, 954-965
- **Wernet et al.** *J Exp Med,* 1973, vol. 138, 1021-1026
- **Takeuchi et al.** *Scand J Immunol,* 1982, vol. 16, 369-377
- **Mimura et al.** *J Exp Med,* 1990, vol. 172, 653-656
- **Czyzyk et al.** *Arthritis Rheum,* 1996, vol. 39, 592-599
- **Revillard et al.** *J Immunol,* 1979, vol. 122, 614-618
- **Proper et al.** *Clin Sci (Lond),* 1991, vol. 80, 87-93
- **Khatlani et al.** *J Immunother,* 2003, vol. 26, 12-20
- **Matsui et al.** *J Immunol,* 1999, vol. 162, 4328-4335
- **Sanz ML ; Prieto I ; Garcia BE ; Oehling A.** Diagnostic reliability considerations of specific IgE determination. *J Invest Allergol Clin Immunol.,* Mai 1996, vol. 6 (3), 152-61
- **Wolthers OD.** Eosinophil granule proteins in the assessment of airway inflammation in pediatric bronchial asthma. *Pediatr Allergy Immunol.,* August 2003, vol. 14 (4), 248-54
- **Lughtenberg, B.** *FEBS Lett.,* 1975, vol. 58, 254
- **Neuber et al.** *Immunology,* 2003, vol. 109, 24-31
- **Linsley et al.** *J Exp Med,* 1991, vol. 174, 561-569

SEQUENZPROTOKOLL

<110> Universitätsklinikum Hamburg-Eppendorf

<120> Diagnose von allergischen Erkrankungen, atopischen Erkrankungen und/oder Autoimmunerkrankungen durch Nachweis von Autoantikörpern gegen CD 28 in humanem Serum

<130> P70674

<160> 5

<170> PatentIn version 3.1

<210> 1
<211> 220
<212> PRT
<213> Homo sapiens

<400> 1

```
Met Leu Arg Leu Leu Leu Ala Leu Asn Leu Phe Pro Ser Ile Gln Val
1               5                   10                  15
Thr Gly Asn Lys Ile Leu Val Lys Gln Ser Pro Met Leu Val Ala Tyr
            20                  25                  30
Asp Asn Ala Val Asn Leu Ser Cys Lys Tyr Ser Tyr Asn Leu Phe Ser
        35                  40                  45
Arg Glu Phe Arg Ala Ser Leu His Lys Gly Leu Asp Ser Ala Val Glu
    50                  55                  60
Val Cys Val Val Tyr Gly Asn Tyr Ser Gln Gln Leu Gln Val Tyr Ser
65                  70                  75                  80
Lys Thr Gly Phe Asn Cys Asp Gly Lys Leu Gly Asn Glu Ser Val Thr
                85                  90                  95
Phe Tyr Leu Gln Asn Leu Tyr Val Asn Gln Thr Asp Ile Tyr Phe Cys
            100                 105                 110
Lys Ile Glu Val Met Tyr Pro Pro Pro Tyr Leu Asp Asn Glu Lys Ser
        115                 120                 125
Asn Gly Thr Ile Ile His Val Lys Gly Lys His Leu Cys Pro Ser Pro
    130                 135                 140
Leu Phe Pro Gly Pro Ser Lys Pro Phe Trp Val Leu Val Val Val Gly
145                 150                 155                 160
Gly Val Leu Ala Cys Tyr Ser Leu Leu Val Thr Val Ala Phe Ile Ile
                165                 170                 175
Phe Trp Val Arg Ser Lys Arg Ser Arg Leu Leu His Ser Asp Tyr Met
            180                 185                 190
Asn Met Thr Pro Arg Arg Pro Gly Pro Thr Arg Lys His Tyr Gln Pro
        195                 200                 205

Tyr Ala Pro Pro Arg Asp Phe Ala Ala Tyr Arg Ser
    210                 215                 220
```

<210> 2
<211> 135
<212> PRT
<213> Homo sapiens

<400> 2

Pro Ser Ile Gln Val Thr Gly Asn Lys Ile Leu Val Lys Gln Ser Pro
1 5 10 15
Met Leu Val Ala Tyr Asp Asn Ala Val Asn Leu Ser Cys Lys Tyr Ser
20 25 30
Tyr Asn Leu Phe Ser Arg Glu Phe Arg Ala Ser Leu His Lys Gly Leu
35 40 45
Asp Ser Ala Val Glu Val Cys Val Val Tyr Gly Asn Tyr Ser Gln Gln
50 55 60
Leu Gln Val Tyr Ser Lys Thr Gly Phe Asn Cys Asp Gly Lys Leu Gly
65 70 75 80
Asn Glu Ser Val Thr Phe Tyr Leu Gln Asn Leu Tyr Val Asn Gln Thr
85 90 95
Asp Ile Tyr Phe Cys Lys Ile Glu Val Met Tyr Pro Pro Pro Tyr Leu
100 105 110
Asp Asn Glu Lys Ser Asn Gly Thr Ile Ile His Val Lys Gly Lys His
115 120 125
Leu Cys Pro Ser Pro Leu Phe
130 135

<210> 3
<211> 3804
<212> DNA
<213> Homo sapiens

<400> 3

taaagtcatc aaaacaacgt tatatcctgt gtgaaatgct gcagtcagga tgccttgtgg 60
tttgagtgcc ttgatcatgt gccctaaggg gatggtggcg gtggtggtgg ccgtggatga 120
cggagactct caggccttgg caggtgcgtc tttcagttcc cctcacactt cgggttcctc 180
ggggaggagg ggctggaacc ctagcccatc gtcaggacaa agatgctcag gctgctcttg 240
gctctcaact tattcccttc aattcaagta acaggaaaca agatttggt gaagcagtcg 300
cccatgcttg tagcgtacga caatgcggtc aaccttagct gcaagtattc ctacaatctc 360

ttctcaaggg agttccgggc atcccttcac aaaggactgg atagtgctgt ggaagtctgt 420
gttgtatatg ggaattactc ccagcagctt caggtttact caaaaacggg gttcaactgt 480
gatgggaaat tgggcaatga atcagtgaca ttctacctcc agaatttgta tgttaaccaa 540
acagatattt acttctgcaa aattgaagtt atgtatcctc ctccttacct agacaatgag 600
aagagcaatg gaaccattat ccatgtgaaa gggaaacacc tttgtccaag tcccctattt 660
cccggacctt ctaagccctt ttgggtgctg gtggtggttg gtggagtcct ggcttgctat 720
agcttgctag taacagtggc ctttattatt ttctgggtga ggagtaagag gagcaggctc 780
ctgcacagtg actacatgaa catgactccc cgccgccccg ggcccacccg caagcattac 840
cagccctatg ccccaccacg cgacttcgca gcctatcgct cctgacacgg acgcctatcc 900
agaagccagc cggctggcag cccccatctg ctcaatatca ctgctctgga taggaaatga 960
ccgccatctc cagccggcca cctcaggccc ctgttgggcc accaatgcca atttttctcg 1020
agtgactaga ccaaatatca agatcatttt gagactctga aatgaagtaa aagagatttc 1080
ctgtgacagg ccaagtctta cagtgccatg gcccacattc caacttacca tgtacttagt 1140
gacttgactg agaagttagg gtagaaaaca aaaagggagt ggattctggg agcctcttcc 1200
ctttctcact cacctgcaca tctcagtcaa gcaaagtgtg gtatccacag acattttagt 1260
tgcagaagaa aggctaggaa atcattcctt ttggttaaat gggtgtttaa tcttttggtt 1320
agtgggttaa acggggtaag ttagagtagg gggagggata ggaagacata tttaaaaacc 1380
attaaaacac tgtctcccac tcatgaaatg agccacgtag ttcctattta atgctgtttt 1440
cctttagttt agaaatacat agacattgtc ttttatgaat tctgatcata tttagtcatt 1500
ttgaccaaat gagggatttg gtcaaatgag ggattccctc aaagcaatat caggtaaacc 1560
aagttgcttt cctcactccc tgtcatgaga cttcagtgtt aatgttcaca atatactttc 1620
gaaagaataa aatagttctc ctacatgaag aaagaatatg tcaggaaata aggtcacttt 1680
atgtcaaaat tatttgagta ctatgggacc tggcgcagtg gctcatgctt gtaatcccag 1740
cactttggga ggccgaggtg ggcagatcac ttgagatcag gaccagcctg gtcaagatgg 1800
tgaaactccg tctgtactaa aaatacaaaa tttagcttgg cctggtggca ggcacctgta 1860
atcccagctg cccaggaggc tgaggcatga gaatcgcttg aacctggcag gcggaggttg 1920
cagtgagccg agatagtgcc acagctctcc agcctgggcg acagagtgag actccatctc 1980
aaacaacaac aacaacaaca acaacaacaa caaaccacaa aattatttga gtactgtgaa 2040
ggattatttg tctaacagtt cattccaatc agaccaggta ggagctttcc tgtttcatat 2100
gtttcagggt tgcacagttg gtctctttaa tgtcggtgtg gagatccaaa gtgggttgtg 2160
gaaagagcgt ccataggaga agtgagaata ctgtgaaaag ggatgttagc attcattaga 2220
gtatgaggat gagtcccaag aaggttcttt ggaaggagga cgaatagaat ggagtaatga 2280
aattcttgcc atgtgctgag gagatagcca gcattaggtg acaatcttcc agaagtggtc 2340
aggcagaagg tgccctggtg agagctcctt tacagggact ttatgtggtt tagggctcag 2400
agctccaaaa ctctgggctc agctgctcct gtaccttgga ggtccattca catgggaaag 2460
tattttggaa tgtgtctttt gaagagagca tcagagttct taagggactg ggtaaggcct 2520
gaccctgaaa tgaccatgga tatttttcta cctacagttt gagtcaacta gaatatgcct 2580
ggggaccttg aagaatgccc ttcagtggcc ctcaccattt gttcatgctt cagttaattc 2640
aggtgttgaa ggagcttagg ttttagaggc acgtagactt ggttcaagtc tcgttagtag 2700
ttgaatagcc tcaggcaagt cactgcccac ctaagatgat ggttcttcaa ctataaatgg 2760
agataatggt tacaaatgtc tcttcctata gtataatctc cataagggca tggcccaagt 2820
ctgtctttga ctctgcctat ccctgacgtt tagtagcatg cccgacatac aatgttagct 2880

```
attggtatta ttgccatata gataaattat gtataaaaat taaactgggc aatagcctaa   2940
gaagggggga atattgtaac acaaatttaa acccactacg cagggatgag gtgctataat   3000
atgaggacct tttaacttcc atcattttcc tgtttcttga aatagtttat cttgtaatga   3060
aatataaggc acctcccact tttatgtata gaaagaggtc ttttaatttt tttttaatgt   3120
gagaaggaag ggaggagtag gaatcttgag attccatatc gaaaatactg tactttggtt   3180
gatttttaag tgggcttcca ttccatggat ttaatcagtc ccaagaagat caaactcagc   3240
agtacttggg tgctgaagaa ctgttggatt taccctggca cgtgtgccac ttgcccagct   3300
tcttgggcac acagagttct tcaatccaag ttatcagatt gtatttgaaa atgacagagc   3360
tggagagttt tttgaaatgg cagtggcaaa taaataaata cttttttta aatggaaaga   3420
cttgatctat ggtaataaat gattttgttt tctgactgga aaaataggcc tactaaagat   3480
gaatcacact tgagatgttt cttactcact ctgcacagaa acaaagaaga aatgttatac   3540
agggaagtcc gttttcacta ttagtatgaa ccaagaaatg gttcaaaaac agtggtagga   3600
gcaatgcttt catagtttca gatatggtag ttatgaagaa aacaatgtca tttgctgcta   3660
ttattgtaag agtcttataa ttaatggtac tcctataatt tttgattgtg agctcaccta   3720
tttgggttaa gcatgccaat ttaaagagac caagtgtatg tacattatgt tctacatatt   3780
cagtgataaa attactaaac tact                                          3804
```

<210> 4
<211> 33
<212> DNA
<213> Artificial

<400> 4
aaagaattcc cttcaattca agtaacagga aac 33

<210> 5
<211> 30
<212> DNA
<213> Artificial

<400> 5
aaacccggga aataggggac ttggacaaag 30